# EUROPEAN PATENT SPECIFICATION

(11) **EP 3 919 607 B1**
(45) Date of publication and mention of the grant of the patent: **15.11.2023**
(21) Application number: 21173198.9
(22) Date of filing: 11.05.2021
(51) Int. Cl.: C12M 1/12, C12M 1/00

(54) **MULTI-COMPARTMENT CONTAINER FOR CULTURING AND SURFACE MULTIPLICATION OF HUMAN AUTOLOGOUS FIBROBLASTS ON A MEMBRANE CARRIER**
MEHRKAMMERBEHÄLTER ZUR KULTIVIERUNG UND OBERFLÄCHENMULTIPLIKATION VON HUMANEN AUTOLOGEN FIBROBLASTEN AUF EINEM MEMBRANTRÄGER
RÉCIPIENT À PLUSIEURS COMPARTIMENTS POUR LA CULTURE DE MULTIPLICATION DE SURFACE DE FIBROBLASTES AUTOLOGUES HUMAINS SUR UN SUPPORT MEMBRANAIRE

(30) Priority: 15.05.2020 PL 43395220
(43) Date of publication of application: 08.12.2021
(73) Proprietor: Slaski Uniwersytet Medyczny w Katowicach, 40-055 Katowice (PL); Uniwersystet Slaski w Katowicach, 40-007 Katowice (PL); Politechnika Slaska, 44-100 Gliwice (PL)
(72) Inventor: Dziedzic, Arkadiusz, 41-902 Bytom (PL); Wojtyczka, Robert, 40-115 Katowice (PL); Kubina, Robert, 40-719 Katowice (PL); Tanasiewicz, Marta, 40-165 Katowice (PL); Skonieczna, Magdalena, 44-103 Gliwice (PL); Talik, Ewa, 40-748 Katowice (PL)
(74) Representative: AOMB Polska Sp. z.o.o.

(56) References cited:
- EP-A1- 0 252 333
- EP-A1- 3 406 703
- CA-A1- 3 093 581
- US-A- 4 066 512
- US-A1- 2010 075 293

## Description

The invention relates to a multi-compartment container for culturing and surface multiplication of human autologous fibroblasts on a membrane carrier in an *in vitro* setting. The container is designed for cytological (cell) culturing of human fibroblasts harvested from a patient and provides an environment suitable for growth thereof. The solution is applicable in particular in alveolar bone surgery.

Fibroblasts are cells found in animals, originate from mesoderm and are the most abundant cells of connective tissue. They have a single round or oval cell nucleus, usually with a distinct nucleolus. Active fibroblasts can be recognised by rough endoplasmic reticulum. Inactive fibroblasts, also called fibrocytes, are smaller and spindle-shaped, with a reduced reticulum. Fibroblasts and fibrocytes are non-circulating, but are able to move. The frequency of mitotic divisions of fibroblasts increases when connective tissue is healing, mediated by the fibroblast growth factor, FGF. Fibroblasts that can undergo mitotic divisions are often referred to as reticular cells. Fibroblasts produce collagen, fibres of the extracellular matrix, proteoglycans of the ground substance [Wojciech Sawicki: Histologic. S. 123-124, PZWL, 2005. ISBN 83-200-3127-3].

Cell culturing allows for multiplying the cells harvested from the human body and keeping them viable. Cell culturing is conducted under special conditions, in the dark, exposed to carbon dioxide, at a temperature of 37°C. Cells for the culture are harvested during medical procedures, surgeries, biopsies, etc. They can be harvested from both healthy and cancerous tissue, depending on specific needs. In recent years, cell cultures have become a widely used tool in research on modern drugs, cosmetics and other substances that affect human cells. They can be used to actively and safely test the effects of particular substances on human tissue; moreover, they allow such testing under strictly controlled experimental conditions. This significantly reduces the costs and time required for testing compared with, for example, animal testing, which, as in case of testing cosmetics, is further banned in the European Union.

Autologous cell culturing conducted in an *in vitro* setting allows for multiplying the cells harvested from an individual patient and keeping them viable. Cell culturing is conducted under special conditions using a cell growth substrate exposed to carbon dioxide at a temperature of 37°C. Human fibroblasts for culturing are harvested for example during minimally invasive medical procedures, surgeries or biopsies of mucosa and skin, i.e. from normal tissues. Fibroblasts are cells located in the mucosa, submucosa and dermis. They produce collagen and elastin, which makes soft tissue stable and elastic while protecting them from mechanical injuries. In the field of oral cavity regeneration procedures, such as periodontal, mucosal and gingival surgery, autologous fibroblasts obtained from an *in vitro* culture have the potential to enhance primary repair and regenerative effects, to accelerate healing, and to stimulate tissue renewal processes. Fibroblasts are a cellular component essential for the success of procedures of mucogingival surgery, recession coverage and augmentation of attached gingiva zone width. Their regenerative potential mediates the process of proper healing and tissue regeneration, as well as the formation of periodontal collagen fibres and soft tissues.

Patent specification US6110208A discloses a method for obtaining artificial skin with a biocompatible layer of fibroblasts cultured on a membrane substrate for allogeneic dermatological grafts. The method consists in seeding a culture of keratinocytes on a microperforated membrane based on a hyaluronic acid derivative and expanding said keratinocytes on said membrane according to conventional techniques, including the use of fetal calf serum, until partial or complete fusion is achieved. Fibroblasts isolated from dermis from other areas are then cultured using conventional techniques in DMEM containing 10% fetal calf serum, conditioning said fibroblasts for proliferation by seeding them on plastic, seeding the fibroblasts in fibrous tissue and continuing the culture until freezing in case the artificial skin obtained has to be preserved or until grafting. The next step involves arranging the previously formed epithelial layer on the fibrous tissue colonised by fibroblasts while making sure that the outer edge of the microperforated membrane does not contact the bottom of the vessel and that it extends slightly beyond the underlying fibrous tissue. Optionally, layers of epithelial cells on a microperforated membrane are fused to the underlying fibrovascular tissue colonised by fibroblasts using biological adhesives selected from the group consisting of collagen, fibrin and fibrin glue. Finally, an aliquot of the same substrate used in the initial step is added, keeping the culture submerged so that the keratinocytes of the top layer of the microperforated membrane are at the air-liquid interface, adding, at the first and subsequent replacements, a medium, i.e. ascorbic acid at a concentration of 1 µg/ml, continuing the culture until grafting or freezing. Weaknesses of the solution according to this invention include the relatively long running time of the method, the need to change the substrate and the transfer of the carrier to a new, fresh substrate.

Patent specification PL209784 discloses a method of primary culture of human fibroblasts for autologous augmentation procedures derived from the connective tissue of the keratinised gingival of the oral mucosa for use in augmentation procedures, i.e. thickening and widening of the soft tissues of the alveolar process. The method consists in fragments of connective tissue of keratinized gingival having an area of 1 to 2 mm², harvested using a known medical technique in a sterile manner, being placed on Petri dishes in a culture fluid supplemented with 10% fetal serum and 0.03% L-glutamine and 100 µg/ml Amphotericin B, followed by the material being mechanically comminuted into pieces having approximately 0.4 mm² in size and culture being conducted for 3 to 5 days under 5% CO₂, while once a complete monolayer is obtained, the cells are transferred onto a carrier, which is a collagen mesh made of highly purified porcine collagen in a three-dimensional form having a shape and surface adapted to the size of the defect in the soft tissue of the keratinised gingival, resuspended in the culture fluid and left for up to 5 days, followed by the carrier with the cells being transferred to the recipient site of the patient. Weaknesses of the solution according to this invention include the relatively long running time of the method and the need for physical transfer of gingival fragments when replacing the liquid substrate medium.

Further, a solution is known relating to a design of a device for culturing fibroblasts ("Cell culture devices having ultrathin porous membrane and uses thereof", US8119394B2), which is used for *in vitro* culture of two or more cell lines on a porous membrane substrate arranged between the culture chambers and allows to detect cell interactions. The device comprises first and second chambers separated by at least one nanoscale membrane disposed between the first and second chambers, wherein at least one nanoscale membrane has an average thickness of less than about 100 nm and has a plurality of pores extending between the opposite sides thereof. It also comprises a cell culture medium in the first and second chambers, a first cell type in the first chamber and a second cell type in the second chamber. The main drawback of this solution is that it provides no possibility for the liquid substrate medium to be continuously transferred between chambers.

Patent specification EP3406703 discloses a device, consisting of three tubular sections and a central diaphragm, a carbon disk, for culturing biofilm forming organisms.

The object of the present inventors was to develop a container for obtaining a hybrid biological component with a collagen carrier ready for use in a clinical setting, in the form of a cellular augmented material, in particular of alveolar bone, with a design that would enable and ensure an accelerated engraftment and regeneration during an alveolar surgical procedure. Another object was to develop a solution that would allow for using the compartments of the container as interchangeable storage for the liquid medium, including a possibility to replace it.

This has been solved according to the invention in a manner, wherein a multi-compartment container for culturing and surface multiplication of human autologous fibroblasts on a membrane carrier consists of three compartments (sections/units) in the form of sleeves with the same diameter, i.e. the bottom compartment, the middle compartment and the top compartment, detachably connected to each other, wherein two transverse shutters are mounted, with the shutter aperture being adjustable from completely closed to open position, wherein one shutter is mounted in the top compartment and the other one at the interface of the bottom and middle compartments separating the inner chambers of those compartments from each other, wherein at the bottom the container is provided with a base forming the closure of the bottom compartment, at the top the container is provided with a closure of the top compartment, and at the interface of the top and middle compartments, namely either in the top compartment at its base, or, more preferably, in the middle compartment at its top edge, a stabilising ring is mounted, on which a sterile collagen membrane made of human or animal collagen for culturing human fibroblasts is securely mounted, for example glued. The ring stabilising the membrane is more preferably arranged at the top edge of the middle compartment, as it is easier to mount it at the top of the middle compartment rather than at the bottom edge of the top compartment.

Preferably, the height of the bottom compartment represents 2/3 of the height of the top compartment, while the height of the middle compartment represents 1/3 of the height of the top compartment, and the shutter in the top compartment is located at 1/3 of the height of this compartment as seen from the base.

Preferably, the stabilising ring for the collagen membrane is mounted so that it can be rotated (together with the membrane mounted thereon) by 360° around a rotation axis perpendicular to the longitudinal axis of the container. Rotation of the ring with the membrane allows for changing the membrane angle, which in turn allows for selective regulation of fibroblast growth in various parts of the membrane.

Preferably, the top closure of the top compartment is a threaded nut.

Preferably, the container is provided with a microbiological filter for preventing microbial contamination, arranged in the upper part of the top compartment, most preferably in the closure of the top compartment, so that it is situated above the liquid cell culturing medium. Microorganisms from the ambient air such as bacteria, viruses and fungi contaminate and infect the medium and may lead to cell death, hence it is expedient to use the filter as above to prevent these microorganisms from contacting the medium.

Preferably, the collagen membrane has a diameter of not more than 40 mm, most preferably between 30 and 40 mm, since above this diameter, after a few days of culture, the membrane may deform or even collapse inwards under the weight of the cultured cells.

Preferably, the height of the middle compartment is not less than 30 mm, which provides adequate space for the medium for the fibroblast culture process.

Preferably, a minimum height of the entire container according to the inventive concept is between 15 cm and 25 cm, depending on the type of cell culture and the biological purpose of the container, i.e. depending on the type of medium, the desired distribution degree of cultured fibroblasts, and the size of the culture membrane.

Preferably, the stabilising ring is made of a polymer material, such as silicone, which provides sufficient flexibility of the component due to the inertia of the material.

Also preferably, the individual compartments of the container are made of plastic, in particular poly(tetrafluoroethylene) (PTFE) or glass. On the other hand, the use of metallic materials that adversely affect cultured cells would be detrimental.

Preferably, a dispensing connector for a pipette is mounted in the closure of the top compartment, which allows for additional substances being added in a sterile manner to the medium in the top compartment during culture, including e.g. substances preventing bacterial or fungal contamination of the medium or agents promoting cell growth (steroids).

The spatial structure of the obtained fibroblast culture depends e.g. on the growth medium and the culture conditions of the cells adapted on a biological membrane. Unlike in classic cell culture, additional components to support cell proliferation, migration and adhesion are required when culturing cells on the surface of collagen membranes. It is therefore important to ensure regular replacement of the medium during cell growth to provide an incubation and metabolic environment, thus preventing nutrient depletion.

The principles of preparing the container for use and of operation of this device according to the invention are described below.

At the interface of the middle and top compartments a stabilising ring is mounted, and the collagen membrane is securely mounted thereon. All three compartments are then connected together, with the device being fitted with two transverse shutters, one at the interface of the bottom and middle compartments and another one in the top compartment.

In order to implement the fibroblast culture process, the closure of the top compartment and the top shutter arranged in the top compartment are opened, while the bottom shutter arranged at the interface of the bottom and middle compartments is closed. Liquid medium with cultured fibroblasts in an amount of the medium not exceeding the capacity of the bottom compartment, preferably equal to the capacity of the bottom compartment, and most preferably filling the space between the bottom and the top shutters, is then added to the container from above and the top shutters is sealed (incomplete filling of said space and leaving empty space may promote the development of microbiological contaminants such as fungi and bacteria in the air). Another portion of fresh fibroblast culture medium, in an amount not exceeding the capacity of the bottom compartment, preferably equal to the capacity of the bottom compartment, is added to the container over the top shutter, followed by the closure of the top compartment, which preferably comprises a microbiological filter, being closed. At this stage, the process of growing fibroblasts on the collagen membrane begins. Additional substances may be added in a sterile manner to the medium during culture via the pipette dosing connector, including e.g. the substances preventing bacterial or fungal contamination of the medium or agents promoting cell growth (steroids).

After a sufficient period of time, preferably between 24 and 48 hours, the bottom shutter is opened and the used medium is removed to the bottom compartment of the container, and the bottom shutter is closed again. The top shutter is then opened, thus introducing a portion of fresh medium into the space between the bottom and the top shutters, followed by the top shutter being closed and allowing it to stay for a period of 48 to 72 hours to continue the culture process in fresh medium, while the bottom compartment is disconnected and the used medium is removed/discharged, and then it is reconnected to the middle compartment. The whole process can be repeated as required.

The solution according to the invention provides the use of a dedicated liquid medium which is a conventional nutrient mixture as a cell growth medium, ensuring stable and undisturbed cell growth without the need for a medium replacement sequence, while allowing homogeneous and atraumatic cell growth on a membrane to be installed in the alveolar bone surgery procedure. As a universal solution, it can be used for Guided Tissue Regeneration (GTR) and Guided Bone Regeneration (GBR), also called augmentation, as well as for studying the structure of the fibroblast monolayer using microscopic techniques. The use of a rotating ring that stabilises the membrane in the container allows for varied, bilateral cell growth on both surfaces of the membrane.

The advantages of the solution include the reduction of the total time spent on the procedure of culturing human fibroblasts on heterogeneous/allogeneous collagen carrier, the reduction of preparatory steps performed by personnel, the atraumatic preparation of the implanted membrane coated with fibroblast monolayer and the reduction of the risk of damage to the fibroblast culture resulting from cell growth on the biological collagen substrate.

Since the container with a circular cross-section preferably has a diameter of up to 40 mm, and taking into account the required volume of the liquid substance for culture that is collected in the container, the minimum height of the entire container according to the inventive concept should range from 15 cm to 25 cm, depending on the type of cell culture and the biological purpose of the container.

Periodic replacement of the medium in each section is regulated by the system of two shutters. Such system ensures stable cultivation of fibroblasts in an *in vitro* setting.

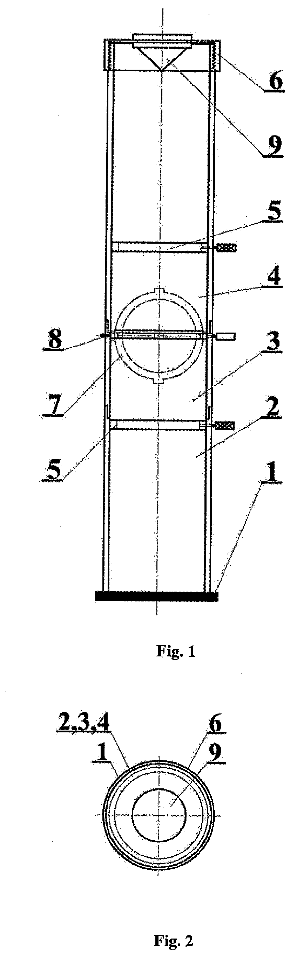

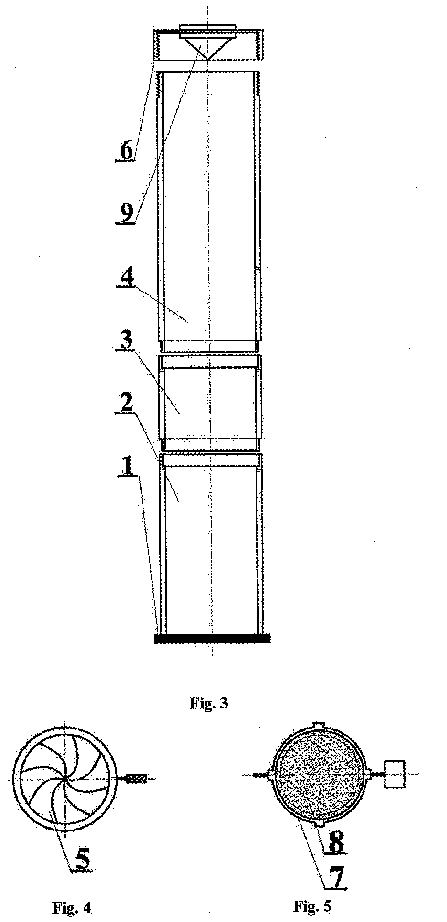

The subject of the invention in an exemplary embodiment is illustrated in the drawing, wherein fig. 1 schematically shows the side view of the container after connecting the individual components, fig. 2 - top view of the container, fig. 3 - side view of the container before connecting the individual components, fig. 4 - top view of the transverse shutter, and fig. 5 - top view of the stabilising ring with collagen membrane mounted thereon.

### Example 1

An exemplary multi-compartment container for culturing and surface multiplication of human autologous fibroblasts on a membrane carrier consists of three compartments (sections/units) in the form of sleeves of equal diameters, that is the bottom compartment (2), the middle compartment (3) and the top compartment (4), detachably connected to each other, wherein two transverse shutters (5) are mounted, with the shutter aperture being adjustable from completely closed to open position, wherein one shutter (5) is mounted in the top compartment (4) and the other one at the interface of the bottom and middle compartments (2,3) separating the inner chambers of those compartments from each other. At the bottom, the container is provided with a base (1) forming the closure of the bottom compartment (2), at the top the container is provided with an closure (6) of the top compartment (4), and at the interface of the top and middle compartments (4, 3), namely in the middle compartment (3) at its top edge a stabilising ring (7) is mounted, on which a sterile collagen membrane (8) made of human or animal collagen for culturing human fibroblasts is securely mounted by gluing.

The bottom compartment (2) is 60 mm high, which represents 2/3 of the top compartment (4) height being 90 mm, while the middle compartment (3) is 30 mm high, which represents 1/3 of the top compartment (4) height, and the shutter (5) in the top compartment (4) is located at 1/3 of this compartment's height as seen from the base (1).

The stabilising ring (7) for the collagen membrane (8) is made of silicone material and is mounted so that it can be rotated (together with the membrane mounted thereon) by 360° around a rotation axis perpendicular to the longitudinal axis of the container. The top closure (6) of the top compartment (4) is a threaded nut which accommodates a microbiological filter to prevent microbial contamination, and a dispensing connector (9) for a pipette is mounted within the closure (6). The collagen membrane (8) has a diameter of 30 mm. The individual compartments (2), (3) and (4) of the container are made of poly(tetrafluoroethylene) (PTFE).

### Example 2

An exemplary multi-compartment container for culturing and surface multiplication of human autologous fibroblasts on a membrane carrier consists of three compartments (sections/units) in the form of sleeves of equal diameters, i.e. the bottom compartment (2), the middle compartment (3) and the top compartment (4), detachably connected to each other, wherein two transverse shutters (5) are mounted, with the shutter aperture being adjustable from completely closed to open position, wherein one shutter (5) is mounted in the top compartment (4) and the other one at the interface of the bottom and middle compartments (2,3) separating the inner chambers of those compartments from each other. At the bottom the container is provided with a base (1) forming the closure of the bottom compartment (2), at the top the container is provided with a closure (6) of the top compartment (4), and at the interface of the top and middle compartments (4, 3), namely in the top compartment (4) at its base a stabilising ring (7) is mounted, on which a sterile collagen membrane (8) made of human or animal collagen for culturing human fibroblasts is securely mounted by gluing.

The bottom compartment (2) is 80 mm high, which represents 2/3 of the top compartment (4) height being 120 mm, while the middle compartment (3) is 40 mm high, which represents 1/3 of the top compartment (4) height, and the shutter (5) in the top compartment (4) is located at 1/3 of this compartment height as seen from the base (1).

The stabilising ring (7) for the collagen membrane (8) is made of polymer material. The top closure (6) of the top compartment (4) is a threaded nut provided with a dispensing connector (9) for a pipette. The collagen membrane (8) has a diameter of 40 mm. The individual compartments (2), (3) and (4) of the container are made of glass.

The solution according to the invention may be used for clinical purposes in periodontal surgery procedures, alveolar surgery (guided tissue regeneration technique) and/or aesthetic surgery. A multi-compartment module for cell culture medium with a membrane carrier for fibroblasts enables efficient and atraumatic acquisition of a layer of autologous fibroblasts on a collagen membrane in a clinical setting for use i.a. in oral cavity regeneration procedures. A heterogeneous, resorbable collagen membrane, usually of porcine origin, enables guided tissue regeneration (GTR) in the periodontal area, providing accelerated regeneration of damaged/resorbed alveolar bone.

### List of numeral references:

- 2: 1- bottom base forming the closure of the bottom compartment - bottom compartment
- 3: - middle compartment
- 4: - top compartment
- 5: - transverse shutters
- 6: - closure of the top compartment (top nut)
- 7: - stabilising ring
- 8: - collagen membrane
- 9: - dosing connector

## Claims

1. A multi-compartment container for culturing and surface multiplication of human autologous fibroblasts on a membrane carrier, **characterised in that** it consists of three compartments in the form of sleeves with the same diameter, i.e. a bottom compartment (2), a middle compartment (3) and a top compartment (4), detachably connected to each other, wherein two transverse shutters (5) are mounted, with the shutter aperture being adjustable from completely closed to open position, wherein one shutter (5) is mounted in the top compartment (4) and the other one at the interface of the bottom and middle compartments (2,3) separating the inner chambers of those compartments from each other, wherein at the bottom the container is provided with a base (1) forming the closure of the bottom compartment (2), at the top the container is provided with a closure (6) of the top compartment (4), and at the interface of the top and middle compartments (4, 3), namely either in the top compartment (4) at its base, or, more preferably, in the middle compartment (3) at its top edge, a stabilising ring (7) is mounted, on which a sterile collagen membrane (8) made of human or animal collagen for culturing human fibroblasts is securely mounted, for example glued.

2. The multi-compartment container according to claim 1, **characterised in that** the height of the bottom compartment (2) represents 2/3 of the height of the top compartment (4), while the height of the middle compartment (3) represents 1/3 of the height of the top compartment (4), and the shutter (5) in the top compartment (4) is located at 1/3 of the height of this compartment as seen from the base (1).

3. The multi-compartment container according to claim 1, **characterised in that** the stabilising ring (7) for the collagen membrane (8) is mounted so that it can be rotated (together with the membrane mounted thereon) by 360° around a rotation axis perpendicular to the longitudinal axis of the container.

4. The multi-compartment container according to claim 1, **characterised in that** the top closure (6) of the top compartment (4) is a threaded nut.

5. The multi-compartment container according to claim 1, **characterised in that** the container is provided with a microbiological filter for preventing microbial contamination, arranged in the upper part of the top compartment (4), most preferably in the closure (6) of the top compartment (4), so that it is situated above the liquid cell culturing medium.

6. The multi-compartment container according to claim 1, **characterised in that** the collagen membrane (8) has a diameter of not more than 40 mm, most preferably between 30 and 40 mm.

7. The multi-compartment container according to claim 1, **characterised in that** the height of the middle compartment (3) is not less than 30 mm.

8. The multi-compartment container according to claim 1, **characterised in that** a minimum height of the entire container is between 15 cm and 25 cm.

9. The multi-compartment container according to claim 1, **characterised in that** the stabilising ring (7) is made of a polymer material, such as silicone.

10. The multi-compartment container according to claim 1, **characterised in that** the individual compartments (2), (3) and (4) of the container are made of plastic, in particular poly(tetrafluoroethylene) (PTFE) or glass.

11. The multi-compartment container according to claim 1, **characterised in that** a dispensing connector (9) for a pipette is mounted in the closure (6) of the top compartment (4).

## Patentansprüche

1. Ein Mehrkammerbehälter zur Kultivierung und Oberflächenvermehrung humaner autologer Fibroblasten auf einem Membranträger, **dadurch gekennzeichnet, dass** er aus drei hülsenförmigen Kammern mit gleichem Durchmesser besteht, nämlich einer unteren Kammer (2), einer mittleren Kammer (3) und einer oberen Kammer (4), lösbar miteinander verbunden, wobei zwei Querverschlussklappen (5) angebracht sind, deren Öffnung von der vollständig geschlossenen in die geöffnete Stellung verstellbar ist, wobei in der oberen Kammer eine Verschlussklappe (5) angebracht ist (4) und die andere an der Schnittstelle der unteren und mittleren Kammern (2,3) und sie die Innenkammern dieser Kammern voneinander trennt, wobei der Behälter an der Unterseite mit einem Boden (1) versehen ist, der den Verschluss der unteren Kammer bildet (2), oben ist der Behälter mit einem Verschluss (6) der oberen Kammer (4) versehen, und zwar an der Schnittstelle der oberen und mittleren Kammern (4, 3), nämlich entweder in der oberen Kammer (4) an ihrem Boden, oder besser noch in der mittleren Kammer (3) an ihrer Oberkante, ist ein Stabilisierungsring (7) angebracht, auf dem eine sterile Kollagenmembran (8) aus menschlichem oder tierischem Kollagen zur Kultivierung menschlicher Fibroblasten sicher befestigt wird, beispielsweise verklebt.

2. Mehrkammerbehälter nach Anspruch 1, **dadurch gekennzeichnet, dass** die Höhe der unteren Kammer (2) 2/3 der Höhe der oberen Kammer (4) beträgt, während die Höhe der mittleren Kammer (3) 1/3 der Höhe der oberen Kammer (4) beträgt, und die Verschlussklappe (5) in der oberen Kammer (4) vom Boden (1) aus gesehen sich auf 1/3 der Höhe dieser Kammer befindet.

3. Mehrkammerbehälter nach Anspruch 1, **dadurch gekennzeichnet, dass** der Stabilisierungsring (7) für die Kollagenmembran (8) 360° um eine senkrechte Drehachse (zusammen mit der darauf montierten Membran) zur Längsachse des Behälters drehbar gelagert ist.

4. Mehrkammerbehälter nach Anspruch 1, **dadurch gekennzeichnet, dass** der obere Verschluss (6) der oberen Kammer (4) eine Gewindemutter ist.

5. Mehrkammerbehälter nach Anspruch 1, **dadurch gekennzeichnet, dass** der Behälter mit einem mikrobiologischen Filter zur Verhinderung einer mikrobiellen Kontamination ausgestattet ist, der im oberen Teil der oberen Kammer (4), am besten im Verschluss (6) der oberen Kammer (4) angeordnet ist, so dass er sich über dem flüssigen Zellkulturmedium befindet.

6. Mehrkammerbehälter nach Anspruch 1, **dadurch gekennzeichnet, dass** die Kollagenmembran (8) einen Durchmesser von nicht mehr als 40 mm, am meisten bevorzugt zwischen 30 und 40 mm, aufweist.

7. Mehrkammerbehälter nach Anspruch 1, **dadurch gekennzeichnet, dass** die Höhe der mittleren Kammer (3) nicht weniger als 30 mm beträgt.

8. Mehrkammerbehälter nach Anspruch 1, **dadurch gekennzeichnet, dass** die Mindesthöhe des gesamten Behälters zwischen 15 cm und 25 cm liegt.

9. Mehrkammerbehälter nach Anspruch 1, **dadurch gekennzeichnet, dass** der Stabilisierungsring (7) aus einem Polymermaterial, beispielsweise Silikon, besteht.

10. Mehrkammerbehälter nach Anspruch 1, **dadurch gekennzeichnet, dass** die einzelnen Kammern (2), (3) und (4) des Behälters aus Kunststoff, insbesondere Poly(tetrafluorethylen) (PTFE) oder Glas bestehen.

11. Mehrkammerbehälter nach Anspruch 1, **dadurch gekennzeichnet, dass** im Verschluss (6) der oberen Kammer (4) ein Dosieranschluss (9) für eine Pipette angebracht ist.

## Revendications

1. Récipient à compartiments multiples pour la culture et la multiplication en surface de fibroblastes autologues humains sur un support membranaire, **caractérisé en ce qu'**il est constitué de trois compartiments en forme de manchons de même diamètre, à savoir: un compartiment inférieur (2), un compartiment intermédiaire (3) et un compartiment supérieur (4), reliés l'un à l'autre de manière amovible, dans lesquels sont montés deux volets transversaux (5), dont l'ouverture est réglable de la position complètement fermée à la position ouverte, un volet (5) étant monté dans le compartiment supérieur (4) et l'autre à l'interface des compartiments inférieur et intermédiaire (2,3), séparant les chambres intérieures de ces compartiments l'une de l'autre, dans lequel le récipient est muni d'une base (1) formant la fermeture du compartiment inférieur (2), d'une fermeture (6) du compartiment supérieur (4) et d'une interface entre les compartiments supérieur et intermédiaire (4, 3), soit dans le compartiment supérieur (4), soit dans le compartiment intermédiaire (4), soit dans le compartiment supérieur (4) à sa base, soit, plus préférablement, dans le compartiment central (3) à son bord supérieur, un anneau de stabilisation (7) est monté, sur lequel une membrane de collagène stérile (8) faite de collagène humain ou animal pour la culture de fibroblastes humains est solidement montée, par exemple collée.

2. Le récipient à compartiments multiples selon la revendication 1, **caractérisé en ce que** la hauteur du compartiment inférieur (2) représente 2/3 de la hauteur du compartiment supérieur (4), tandis que la hauteur du compartiment intermédiaire (3) représente 1/3 de la hauteur du compartiment supérieur (4), et que l'obturateur (5) du compartiment supérieur (4) est situé à 1/3 de la hauteur de ce compartiment, vu de la base (1).

3. Le conteneur à compartiments multiples selon la revendication 1, **caractérisé en ce que** l'anneau de stabilisation (7) de la membrane de collagène (8) est monté de manière à pouvoir tourner (avec la membrane montée dessus) de 360° autour d'un axe de rotation perpendiculaire à l'axe longitudinal du conteneur.

4. Le récipient à compartiments multiples selon la revendication 1, **caractérisé en ce que** la fermeture supérieure (6) du compartiment supérieur (4) est un écrou fileté.

5. Le récipient à compartiments multiples selon la revendication 1, **caractérisé en ce que** le récipient est pourvu d'un filtre microbiologique pour prévenir la contamination microbienne, disposé dans la partie supérieure du compartiment supérieur (4), de préférence dans la fermeture (6) du compartiment supérieur (4), de sorte qu'il est situé au-dessus du milieu liquide de culture cellulaire.

6. Le récipient à compartiments multiples selon la revendication 1, **caractérisé en ce que** la membrane de collagène (8) a un diamètre inférieur ou égal à 40 mm, de préférence compris entre 30 et 40 mm.

7. Le récipient à compartiments multiples selon la revendication 1, **caractérisé en ce que** la hauteur du compartiment central (3) n'est pas inférieure à 30 mm.

8. Le récipient à compartiments multiples selon la revendication 1, **caractérisé en ce que** la hauteur minimale de l'ensemble du récipient est comprise entre 15 et 25 cm.

9. Le récipient à compartiments multiples selon la revendication 1, **caractérisé en ce que** l'anneau stabilisateur (7) est constitué d'un matériau polymère, tel que le silicone.

10. Le récipient à compartiments multiples selon la revendication 1, **caractérisé en ce que** les compartiments individuels (2), (3) et (4) du récipient sont en plastique, en particulier en poly(tétrafluoroéthylène) (PTFE) ou en verre.

11. Le récipient à compartiments multiples selon la revendication 1, **caractérisé par le fait qu'**un connecteur de distribution (9) pour une pipette est monté dans la fermeture (6) du compartiment supérieur (4).
